# FASCICULE DE BREVET EUROPEEN

(11) **EP 3 166 424 B1**
(45) Date de publication et mention de la délivrance du brevet: **13.11.2019**
(21) Numéro de dépôt: 15748285.2
(22) Date de dépôt: 10.07.2015
(51) Int. Cl.: A24B 15/16

(54) **UTILISATION D'UNE COMPOSITION RENFERMANT UN POLYOL À CHAÎNE LONGUE COMME BASE DE E-LIQUIDES**
VERWENDUNG EINER ZUSAMMENSETZUNG MIT EINEM LANGKETTIGEN POLYOL ALS BASIS FÜR E-FLÜSSIGKEITEN
USE OF A COMPOSITION CONTAINING A LONG-CHAIN POLYOL AS A BASE FOR E-LIQUIDS

(30) Priorité: 10.07.2014 FR 1456655
(43) Date de publication de la demande: 17.05.2017
(73) Titulaire: Laboratoires Ceres, 86000 Poitiers (FR)
(72) Inventeur: PICCIRILLI, Antoine, 86000 Poitiers (FR); BONNARME, Vincent, 36300 Saint-Aigny (FR)
(74) Mandataire: Tripoz, Inès
(86) Numéro de dépôt international: PCT/FR2015/051919
(87) Numéro de publication internationale: WO 2016/005709

(56) Documents cités:
- WO-A1-2013/088230
- CN-A- 103 519 348
- GB-A- 2 469 850
- KR-A- 20100 028 182
- US-A1- 2014 166 027

## Description

### DOMAINE TECHNIQUE

La présente invention se rapporte à l'utilisation d'une composition renfermant un polyol à chaîne longue comme liquide de cigarette électronique. Elle a également pour objet une composition de liquide pour cigarette électronique comprenant un polyol, ainsi que de la nicotine et/ou au moins un arôme, ainsi qu'une cigarette électronique ou un dispositif apparenté renfermant cette composition.

### ARRIERE-PLAN DE L'INVENTION

Le marché de la cigarette électronique connait actuellement un développement important, du fait qu'elle permet au consommateur de conserver le rituel associé à l'emploi de la cigarette sans subir les effets délétères des substances nocives que celle-ci renferme.

La cigarette électronique ou e-cigarette fonctionne à l'électricité sans combustion. Elle produit un brouillard de fines particules, appelé communément vapeur ou fumée artificielle, ressemblant visuellement à la fumée produite par la combustion du tabac. Cette vapeur peut être aromatisée (arome de tabac, de menthe, de fruits, de chocolat, etc.) et contenir ou non de la nicotine. Dans les e-cigarettes correctement fabriquées et utilisées, l'aérosol contient, selon les données disponibles, beaucoup moins de substances délétères à la santé que la fumée du tabac, en particulier ni particules solides, ni goudron, ni autres substances cancérogènes, ni monoxyde de carbone (CO).

L'e-cigarette comporte trois parties principales contenues dans une enveloppe plastique ou métallique :
- une pile,
- une cartouche ou réservoir contenant un liquide dit "e-liquide", et
- un atomiseur.

La pile constitue la plupart du temps la plus grande partie de l'e-cigarette sur les produits jetables. Sur les cigarettes réutilisables, il s'agit de batteries « basse tension » (accumulateurs), rechargeables par câble USB ou par chargeur. Dans les e-cigarettes réutilisables, le tube abritant la pile se visse sur la cartouche contenant le liquide. Sur certains modèles, un voyant lumineux - usuellement une diode rouge ou bleue - est placée à l'autre extrémité du tube de la pile.

Le dispositif de stockage du e-liquide peut prendre la forme d'une cartouche (généralement en silicone, PMMA ou métal inoxydable) ou d'un réservoir (en particulier en PMMA/polyéthylène, verre borosilicate ou métal inoxydable) éventuellement complété d'un dispositif de captation du liquide par capillarité (notamment en silice, fibre de verre, tissu métallique céramique, fils de nylon ou fibres de borosilicate) en contact avec le système de vaporisation. L'atomiseur permet de convertir l'e-liquide en brouillard simulant la fumée. Il est constitué d'une spirale ou treillis métallique qui forme une résistance chauffante. Il est de plus en plus souvent intégré dans la cartouche rechargeable. Une micro-valve sensible à la dépression provoquée par l'inspiration ou un contacteur à déclenchement manuel permettent l'alimentation par la pile de l'atomiseur. L'e-cigarette peut être à usage unique ou réutilisable.

Les e-liquides utilisés sont principalement composés des constituants suivants :
- propylène glycol synthétique (environ 65 %)
- glycérol (environ 25%)
- eau (5 à 10%)
- arômes et colorants (2 à 5%)
- nicotine (0 à 20 mg/ml)
Certains e-liquides peuvent aussi contenir de l'éthanol en quantité significative (>1%).

Certains produits peuvent être dépourvus de propylène glycol synthétique. L'objectif est dans ce cas de pouvoir revendiquer des produits d'origine exclusivement végétale. Cet objectif est toutefois atteint au détriment de la longévité des résistances chauffantes, qui s'encrassent très rapidement. En outre, la qualité de la fumée émise est loin d'être convenable en terme de densité de vapeur, et les propriétés organoleptiques des liquides sont fortement modifiées, car la libération des arômes en l'absence de propylène glycol est rendue moins immédiate et profondément modifiée au plan organoleptique. Par ailleurs, l'emploi exclusif de glycérol force à charger le produit en eau, afin de diminuer la viscosité du e-liquide et de faciliter ainsi le remplissage de la e-cigarette. Mais, là encore, l'impact d'une forte teneur en eau modifie radicalement la qualité de la vapeur émise et conduit à une corrosion excessive des matériaux ainsi qu'à une consommation rapide et excessive du e-liquide (vaporisation plus rapide). En outre, la présence d'eau impose au plan industriel de prendre des précautions drastiques afin d'éviter toute pollution microbiologique (ex. filtration stérile, ajout de conservateur). Enfin, une autre problématique liée à l'usage exclusif de glycérol réside dans le fait que ce composé est nettement moins vaporisable que le propylène glycol, de sorte que sa vaporisation nécessite une température de chauffe nettement plus importante, susceptible d'entraîner sa dégradation et la formation de sous-produits indésirables tels que l'acroléine.

Par conséquent, l'usage du propylène glycol synthétique en quantité plus importante que le glycérol est le plus souvent préféré, ce qui n'autorise pas les fabricants à revendiquer une origine naturelle de leurs produits. De plus, le propylène glycol est obtenu selon un procédé qui compte parmi les plus énergivores de la pétrochimie et de fait affiche une forte empreinte environnementale (Eissen & coll, Angew. Chem. Int. Ed. 2002, 41, 414-436) qui se traduit par une grande consommation d'énergie et une production importante de composés organiques volatils (COVs) et de déchets. En outre, le propylène glycol synthétique est obtenu à partir d'oxyde de propylène selon un procédé continu d'hydratation, selon le schéma suivant :

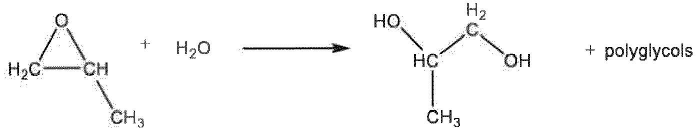

La production de propylène glycol s'accompagne de la formation de produits secondaires (di-, et tri- et tétrapropylène glycols) et d'oxyde de propylène non converti (Petrochemical Processes: Major Oxygenated, Chlorinated and Nitrated Derivatives - Alain Chauvel, Gilles Lefebvre - Editions TECHNIP - p26*),* come illustré ci-dessous :

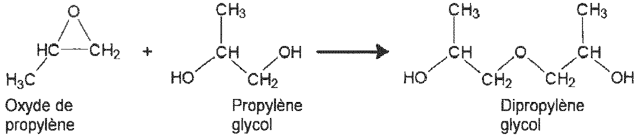

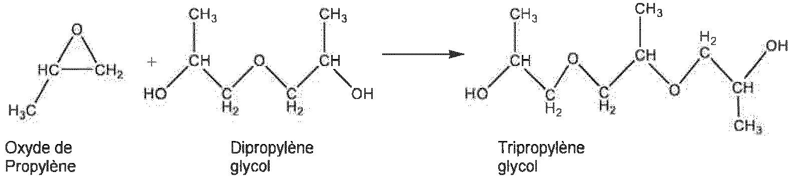

Par conséquent, après purification, les impuretés organiques mineures et récurrentes du propylène glycol sont le di- et tripropylène glycol, ainsi que l'oxyde de propylène dont la teneur résiduelle d'après les producteurs est de l'ordre de 5 à 10 ppm (Propylene Glycol - CIR Expert Panel, June 28-29 2010 - Draft Report). Or, l'oxyde de propylène est classé par les agences environnementales nord-américaine et européenne comme un composé carcinogène et mutagène chez l'animal et comme carcinogène probable chez l'homme. Par conséquent, il appartient de limiter fortement l'exposition à ce composé. Aussi, le Rapport et Avis d'Experts sur l'e-Cigarette publié par l'Office Français de Prévention du Tabagisme (OFT) en mai 2013 insiste sur la nécessité de garantir l'absence de contaminants cancérigènes dans les e-liquides. De fait, il appartient d'éviter la présence d'un composé toxique tel que l'oxyde de propylène et dans une moindre mesure la présence d'impuretés organiques appartenant à la famille fort décriée au plan toxicologique des éthers de glycols, lesquels altèrent la qualité des e-liquides, à l'instar des di- et tripropylène glycols.

Une solution aux problèmes précités a été proposée dans la demande WO 2013/088230. Elle consiste à substituer au propylène glycol synthétique du propylène glycol d'origine végétale, obtenu par hydrogénation catalytique de sorbitol, issu lui-même du maïs. Le propylène glycol est associé à du glycérol d'origine végétale, à de la nicotine qui peut être extraite de feuilles de tabac et éventuellement à des arômes d'origine naturelle, pour obtenir un e-liquide d'origine entièrement végétale.

Si cette solution permet effectivement de s'affranchir des inconvénients liés à l'utilisation de propylène glycol synthétique, il a été mis en évidence que la densité de vapeur et la puissance aromatique produits par ces e-liquides d'origine végétale pouvaient être améliorées en remplaçant le propylène glycol par un polyol à chaîne longue et que cet effet était particulièrement marqué en l'absence de glycérine ou dans une composition de e-liquide à faible teneur en glycérine. En permettant de s'affranchir du glycérol, l'utilisation d'un polyol à chaîne longue contribue en outre à protéger le dispositif chauffant des cigarettes électroniques en supprimant le phénomène d'encrassement rapide observé en présence de glycérine. Un autre avantage lié à l'absence de glycérine est que la vapeur produite se trouve débarrassée des impuretés toxiques et cancérigènes issues de la décomposition thermique du glycérol.
Un autre avantage de l'utilisation d'un polyol à chaîne longue est qu'il permet de formuler la nicotine en absence d'eau. Il est en effet connu que la nicotine base en solution dans l'eau se transforme en nicotine protonée. Or, la forme protonée est nettement moins bio-assimilable que la nicotine base. Par conséquent, l'usage d'un polyol à chaîne longue en absence d'eau permet de concevoir des e-liquides plus performants en terme de sevrage tabagique et plus sécurisés car moins concentrés en nicotine, alcaloïde dont la toxicité aiguë est très élevée. Dans la demande CN103519348, il est divulgué une composition comprenant du « butanediol » (sans précision s'agissant de la position des hydroxyles), de l'eau, ainsi que d'autres ingrédients.
Il a en outre été observé qu'un polyol à chaîne longue permettait d'obtenir des e-liquides dépourvus de nicotine recréant le picotement de la gorge (ou "throat hit") typiquement ressenti par l'utilisateur d'une cigarette classique, lors du passage de la nicotine dans la bouche. Jusqu'à présent, cet effet très recherché par les utilisateurs de cigarettes électroniques n'était obtenu qu'en ajoutant au e-liquide quelques gouttes d'un produit à base de propylène glycol, de glycérine et d'arômes (E-Liquide Flash® de FLAVOUR ART). Toutefois, ce dernier présente tous les inconvénients mentionnés ci-dessus, liés à l'emploi de propylène glycol et de glycérine.
Les polyols à chaîne longue sont principalement d'origine synthétique mais certains sont d'origine renouvelable à l'instar du 1,4 butanediol, du 2,3 butanediol, du 1,2 pentanediol.

La présente invention a pour objet une composition de liquide pour cigarette électronique comprenant un polyol en C4-C8 ayant 2 fonctions hydroxyles, ainsi qu'au moins un composé choisi parmi la nicotine, un substitut de nicotine et un arôme, et ne renfermant pas d'eau.

Elle a encore pour objet une cigarette électronique renfermant cette composition.

Elle a pour objet l'utilisation d'un polyol en C4-C8 ayant 2 groupes hydroxyles dans un liquide de cigarette électronique renfermant ou non de la nicotine, pour améliorer le picotement de la gorge ressenti par un utilisateur dudit liquide et/ou la facilité d'aspiration de la vapeur produite par ledit liquide.

Elle a également pour objet l'utilisation d'un polyol en C4-C8 ayant 2 groupes hydroxyles dans un liquide de cigarette électronique pour renforcer la puissance aromatique ou pour limiter ou pour supprimer la formation de coproduits de thermolyse ou pour augmenter la densité de vapeur.

Elle a enfin pour objet l'utilisation d'un polyol en C4-C8 ayant 2 groupes hydroxyles dans un liquide de cigarette électronique renfermant de la nicotine, pour améliorer la biodisponibilité de la nicotine.

### DESCRIPTION DETAILLEE DE MODES DE REALISATION

Dans la présente demande, on désigne par "cigarette électronique" l'ensemble des dispositifs équipés de moyens électriques produisant de la vapeur et délivrant de la nicotine et/ou un arôme. Cette définition englobe donc notamment les vaporisateurs personnels (VP), les systèmes électroniques de distribution de nicotine (soit ENDS pour "Electronic Nicotine Delivery System", soit ENDD pour "Electronic Nicotine Delivery Device"), ainsi que les cigares électroniques, pipes électroniques et chichas électroniques, cigarettes à base de tabac chauffé ou contenant un arôme de tabac obtenu par macération.

On entend par composé "d'origine végétale" un composé comprenant au moins 95% de carbone biosourcé, tel que déterminé par la norme ASTM D6866 - 12 (*Standard Test Methods for Determining the Biobased Content of Solid, Liquid, and Gaseous Samples Using Radiocarbon Analysis*).

Comme indiqué précédemment, l'invention porte sur l'utilisation d'une composition renfermant un polyol comportant 4 à 8 atomes de carbone ayant 2 fonctions hydroxyles et ne renfermant pas d'eau comme liquide de cigarette électronique (ci-après, "e-liquide").

De plus, comme indiqué précédemment, l'invention porte également sur :
- l'utilisation d'un polyol en C4-C8 ayant 2 groupes hydroxyles dans un liquide de cigarette électronique renfermant ou non de la nicotine, pour améliorer le picotement de la gorge ressenti par un utilisateur dudit liquide et/ou la facilité d'aspiration de la vapeur produite par ledit liquide, et
- l'utilisation d'un polyol en C4-C8 ayant 2 groupes hydroxyles dans un liquide de cigarette électronique pour renforcer la puissance aromatique ou pour limiter ou pour supprimer la formation de coproduits de thermolyse ou pour augmenter la densité de vapeur.

Dans la présente invention, on entend par « polyol comportant 4 à 8 atomes de carbone et 2 fonctions hydroxyles », un composé comportant 4 à 8 atomes de carbone, de préférence 4, 5, 6, 7 ou 8 atomes de carbone et 2 fonctions hydroxyles (-OH).

Dans la présente invention, on entend par polyol à chaîne longue un composé répondant à l'une au moins des caractéristiques suivantes :
- composé hydrocarboné comportant 2, 3 ou 4 fonctions hydroxyles, de préférence 2 ;
- composé comportant 4 à 12 atomes de carbone, de préférence 4, 5, 6, 7, 8, 9 ou 10 atomes de carbone;
- composé linéaire ou cyclique, de préférence linéaire ;
- composé d'origine végétale ou synthétique, de préférence végétale.

Le polyol comportant 4 à 8 atomes de carbone et 2 fonctions hydroxyles ou le polyol à chaîne longue peut être synthétique ou, selon une forme d'exécution préférée de l'invention, il peut être obtenu à partir de matières premières végétales et désigné ici par "polyol d'origine végétale".

De façon non limitative le polyol en C4-C8 ayant 2 groupes hydroxyle selon l'invention appartient au groupe constitué par le 1, 2-butanediol, le 1,3-butanediol, le 1,4-butanediol, le 2-méthyl-1,3-propanediol, le 2-méthyl-1,3-butanediol, le 2-méthyl-1,4-butanediol, le 2-éthyl-1,3-propanediol, le 1,2-pentanediol, le 1,3-pentanediol, le 1,4-pentanediol, le 1,5-pentanediol, le 1,2-hexanediol, le 1,3-hexanediol, le 1,4-hexanediol, le 1,5-hexanediol, le 1,6-hexanediol, le 2-méthyl-1,3-pentanediol, 2-méthyl-1,4-pentanediol, le 2-méthyl-1,5-pentanediol, le 2-éthyl-1,3-butanediol, le 2-éthyl-1,4-butanediol, le 1,2-octanediol, le 1,3-octanediol, le 1,4-octanediol, le 1,5-octanediol, le 1,6-octanediol, le 1,7-octanediol, le 1,8-octanediol, le 2-méthyl-1,3-heptanediol, le 2-méthyl-1,4-heptanediol, le 2-méthyl-1,5-heptanediol, le 2-méthyl-1,6-heptanediol, le 2-méthyl-1,7-heptanediol, le 3-méthyl-1,2-heptanediol, le 3-méthyl-1,3-heptanediol, le 3-méthyl-1,4-heptanediol, le 3-méthyl-1,5-heptanediol, le 3-méthyl-1,6-heptanediol, le 3-méthyl-1,7-heptanediol, le 2-éthyl-1,3-hexanediol, le 2-éthyl-1,4-hexanediol, le 2-éthyl-1,5-hexanediol, le 2-éthyl-1,6-hexanediol, le 3-éthyl-1,2-hexanediol, le 3-éthyl-1,3-hexanediol, le 3-éthyl-1,4-hexanediol, le 3-éthyl-1,5-hexanediol, le 3-éthyl-1,6-hexanediol, le 1,4-cyclohexanediol et le méthyl-2-1,4-cyclohexanediol.

De préférence le polyol est le 1,2-butanediol, le 1,3-butanediol, le 1,4-butanediol, le 1,2-pentanediol, le 1,2-hexanediol, le 1,2-heptanediol ou le 1,2-octanediol.

La composition utilisée selon l'invention peut renfermer en outre du propylène glycol. Ce dernier peut être d'origine synthétique ou végétale (c'est-à-dire obtenu à partir de matières premières végétales). Dans ce dernier cas, qui est préféré, le propylène glycol peut en particulier être obtenu par hydrogénolyse de sorbitol ou de glycérol végétal (New and Future Developments in Catalysis : Catalytic Biomass Chemistry - S. Suib Editor/Elsevier - 2013, pp 13-17*)* ou par hydrogénation d'acide lactique végétal (J. Van Haveren & coll. Bulk Chemicals from Biomass. BioFPR, november 1, 2007. pp 41-57).

Le glycérol biosourcé mis en œuvre pour produire le propylène glycol peut être d'origine animale ou végétale, de préférence végétale. Le glycérol végétal est issu de l'hydrolyse (acide ou basique) des huiles végétales ou de leur alcoolyse (transestérification). Ces huiles appartiennent de façon non limitative au groupe des huiles de soja, de palme, de palmiste, de coprah, de colza, de tournesol, de germe de maïs, de coton, d'olive, de sésame, de son de riz, de lin, de ricin, d'avocat, d'arachide, de carthame, de pépins de raisin, ou de pin (tall oil). On préfère utiliser du glycérol issu de variétés végétales non génétiquement modifiées, telles que les huiles de palme, de colza, de tournesol, ou de coprah.

Le sorbitol ou l'acide lactique biosourcé mis en œuvre pour produire le propylène glycol d'origine végétale est généralement issu de plantes sucrières ou amylacées telles que la canne à sucre, le maïs, le blé, la pomme de terre, la betterave sucrière, le riz, ou le sorgho. De préférence, on utilise le sorbitol ou l'acide lactique issu de variétés végétales non génétiquement modifiées, telles que la canne à sucre ou la betterave. Mieux encore, le sorbitol ou l'acide lactique est obtenu à partir de biomasses lignocellulosiques non alimentaires telles que le bois, la paille, les drèches de palmier (palm bunchees), la bagasse, et les rafles de maïs non génétiquement modifié.

On notera que les procédés précités permettent d'obtenir non seulement du propylène glycol, mais également du 1,3-propanediol (PDO) comme co-produit, dont les proportions peuvent être ajustées en choisissant de façon appropriée les conditions de la réaction (Nur Dyana bt Saar - Dissertation submitted in partial fulfillment of the requirements for the Bachelor of Engineering (Hons) Chemical Engineering, Universiti Teknologi PETRONAS, Mai 2013*)* .

Le propylène glycol peut représenter de 2 à 50% en poids, de préférence de 10 à 40% en poids, plus préférentiellement de 20 à 30% en poids, par rapport au poids total de la composition.

On préfère selon l'invention que la composition utilisée comme e-liquide ne contienne pas ou peu de glycérine, c'est-à-dire qu'elle renferme de 0 à 40% en poids de glycérine, de préférence de 0 à 20% en poids, par exemple de 0 à 5% en poids de glycérine ou de 5 à 20% en poids de glycérine, par rapport au poids total de la composition. Il a en effet été observé, comme indiqué plus haut, que l'absence de glycérine permettait d'éviter la formation de sous-produits indésirables lors du chauffage de la glycérine. Il a précisément été constaté qu'à la température atteinte par la résistance d'une cigarette électronique, le glycérol se décomposait en acroléine (Cordoba & coll. Proceedings of COBEM 2011 - 21st Brazilian Congress of Mechanical Engineering, 24-28 octobre 2011, Natal, RN, Brazil Metzger Brian *;* Glycerol Combustion - Thèse de l'Université de Caroline du Nord, 1er Août 2007), composé hautement toxique à très faible concentration (Goniewicz & coll. Levels of Selected Carcinogens and Toxicants in Vapour from Electronic Cigarettes - TC Online First, publié le 6 Mars 2013 sous 10.1136/tobaccocontrol-2012-050859). De façon tout-à-fait surprenante, il a également été noté que l'absence de glycérine permettait d'augmenter nettement la densité de vapeur et la puissance aromatique de la cigarette électronique. Avantageusement, lorsqu'elle est présente, la glycérine est d'origine végétale et obtenue suivant les procédés décrits précédemment.
Par puissance aromatique d'un produit, on entend la perception olfacto-gustative du produit, c'est-à-dire sa flaveur, terme qui regroupe la saveur, l'astringence, la pseudo-chaleur et l'arôme du produit. La puissance aromatique est évaluée selon des protocoles olfacto-gustatifs par un panel de testeurs.

La composition peut aussi contenir du 1,3-propanediol synthétique ou, selon une forme d'exécution préférée de l'invention, il peut être obtenu à partir de matières premières végétales et désigné ici par "1,3-biopropanediol d'origine végétale". Le 1,3-biopropanediol d'origine végétale peut être obtenu par fermentation de glucose, en présence d'une bactérie native ou modifiée génétiquement, choisie notamment parmi les souches de *Klebsiella* (notamment *pneumoniae*), *Clostridium* (notamment *butyricum*), *Citrobacter* (notamment *freundii*), *Serratia* et *Escherichia coli,* de préférence *Escherichia coli,* et plus préférentiellement *Escherichia coli* K-12. Un exemple de souche génétiquement modifiée est décrit dans la demande US 2012/258521. Le glucose biosourcé mis en œuvre pour produire le 1,3-propanediol est généralement issu de plantes sucrières ou amylacées telles que la canne à sucre, le maïs, le blé, la pomme de terre, la betterave sucrière, le riz, ou le sorgho. De préférence, le glucose est issu de variétés végétales non génétiquement modifiées, telles que la canne à sucre ou la betterave. Mieux encore, le glucose est issu de biomasses lignocellulosiques non alimentaires telles que le bois, la paille, les drèches de palmier (palm bunchees), la bagasse, et les rafles de maïs non génétiquement modifié. Le produit de la fermentation peut être récupéré, et le 1,3-biopropanediol purifié, par filtration membranaire, électrodialyse, concentration ou rectification, par exemple, ou par une combinaison de ces techniques. Le 1,3-biopropanediol peut en particulier être purifié par distillation, opération qui permet d'atteindre une pureté de 99,8%. Les impuretés présentes à hauteur de 0,2% sont l'eau et le propanol-1 (Chatterjee & coll. Glycerol to Propylene Glycol /Department of Chemical & Biomolecular Engineering Senior Design Reports (CBE), University of Pensylvania - April 12, 2011), composé dénué de toxicité. Le 1,3-propanediol peut représenter de 2 à 50% en poids, de préférence de 10 à 40% en poids, plus préférentiellement de 20 à 30% en poids, par rapport au poids total de la composition.

Mis à part les constituants précités, la composition utilisée selon l'invention renferme en outre au moins un composé choisi parmi la nicotine, un substitut de nicotine (typiquement une molécule non addictive mais avec un effet sensoriel proche de celui de la nicotine) et un arôme.

La nicotine peut être d'origine synthétique ou végétale et doit répondre de préférence aux critères de pureté décrits dans les pharmacopées américaine (USP) et européenne (PE) en vigueur. Elle peut être notamment extraite de feuilles de tabac ou obtenue par synthèse chimique. La concentration en nicotine dans la composition selon l'invention peut aller de 0 à 100 mg/ml, de préférence de 2 à 25 mg/ml.

Les arômes peuvent également être des arômes d'origine végétale ou synthétique tels que ceux homologués dans les domaines alimentaire et/ou pharmaceutique, en particulier ceux listés dans le règlement UE n° 872/2012 du 1^{er} octobre 2012 et dans les pharmacopées américaine (USP) et européenne (PE) en vigueur. La concentration en arômes peut aller de 0 à 30% en poids, de préférence de 1 à 8% en poids, plus préférentiellement de 2 à 5% en poids, par rapport au poids total de la composition.

La composition utilisée selon l'invention peut également comprendre un alcool tel que l'éthanol et/ou au moins un colorant. L'alcool peut représenter de 0 à 20% en poids, de préférence de 1 à 10% en poids, par rapport au poids total de la composition. Les colorants peuvent être des colorants d'origine végétale ou synthétique, tels que ceux homologués dans les domaines alimentaire et/ou pharmaceutique et en particulier ceux listés dans le règlement UE n° 1331/2008 et dans les pharmacopées américaine (USP) et européenne (PE) en vigueur. La concentration en arômes peut aller de 0 à 30% en poids, de préférence de 1 à 8% en poids, plus préférentiellement de 2 à 5% en poids, par rapport au poids total de la composition.

La composition selon l'invention ne comprend pas d'eau, à l'exception de celle éventuellement contenue dans les matières premières que la composition renferme. En effet, l'eau peut favoriser le développement de microorganismes pathogènes d'origine microbienne et son utilisation nécessite généralement l'emploi de conservateurs ou la réalisation d'une microfiltration stérilisante. Par ailleurs, l'ajout d'eau aux e-liquides induit une transformation de la nicotine base en nicotine protonée. Or, il est connu de l'homme du métier que la forme protonée de la nicotine est nettement moins bio-assimilable, et de fait moins addictive, que la nicotine base. Ainsi, certains polyols à longue chaîne permettent de formuler des e-liquides très fluides, sans avoir à ajouter de l'eau, dans lesquels la nicotine est présente sous forme base et sous sa forme hautement biodisponible, ce qui améliore nettement le contrôle de la délivrance de la nicotine, en particulier lors du sevrage tabagique.

L'invention porte également sur une cigarette électronique renfermant la composition telle que décrite ci-dessus. Celle-ci est généralement disposée dans une cartouche solidarisée à un réceptacle abritant un système d'alimentation électrique relié à un dispositif d'atomisation de la composition.

Elle porte encore sur l'utilisation d'un polyol en C4-C8 ayant 2 fonctions hydroxyles dans un liquide de cigarette électronique renfermant ou non de la nicotine, pour améliorer le picotement de la gorge ressenti par un utilisateur de ladite cigarette électronique et/ou la facilité d'aspiration de la vapeur produite par ledit liquide.
L'invention concerne également l'utilisation d'un polyol en C4-C8 ayant 2 fonctions hydroxyles dans un liquide de cigarette électronique pour renforcer la puissance aromatique.

Elle concerne également l'utilisation d'un polyol en C4-C8 ayant 2 fonctions hydroxyles dans un liquide de cigarette électronique pour limiter ou pour supprimer la formation de coproduits de thermolyse.

Enfin, elle porte sur l'utilisation d'un polyol en C4-C8 ayant 2 fonctions hydroxyles dans un liquide de cigarette électronique pour augmenter la densité de vapeur.

Selon un mode de réalisation, le polyol à chaîne longue est le 1,2-butanediol, le 1,3-butanediol, le 1,4-butanediol, le 1,2-pentanediol, le 1,2-hexanediol, le 1,2-heptanediol ou le 1,2-octanediol.

Elle porte par ailleurs sur l'utilisation d'un polyol comportant 4 à 8 atomes de carbone ayant 2 fonctions hydroxyles dans un liquide de cigarette électronique renfermant de la nicotine, pour améliorer la biodisponibilité de la nicotine.

L'invention sera mieux comprise à la lumière des exemples suivants, qui ne sont donnés qu'à titre purement illustratif et n'ont pas pour but de limiter la portée de l'invention, définie par les revendications annexées.

### EXEMPLES

### Exemple 1 : Préparation et analyse d'une composition à base de 1,3-butanediol et de glycérine

Dans un mélangeur en verre équipé d'une agitation mécanique on mélange précisément 10,00 kg de 1,3-butanediol tel que commercialisé par la société Kyowa sous le nom 1,3-butylène glycol, (grade cosmétique), 1,00 kg de glycérine végétale (commercialisée par la société Oléon sous la référence Glycérine 4810, grade Pharmacopées USP et PE), 450,0 g d'arôme fruité de pomme (commercialisé par la société Safisis sous la référence PT 128) et 114,50 g de nicotine végétale (commercialisée par la société Alchem International sous la référence Nicotine Free Base, Pharmaceutical grade Nicotine >99%). On maintient l'agitation du mélange (50 tours/minute) pendant 20 minutes. Un prélèvement de 500 g est effectué pour analyse. On effectue alors une mesure de la viscosité cinématique à 25°C à l'aide d'un viscosimètre de Houillon
- tube m 943 avec un coefficient de 0,35051

### Résultats :

La viscosité à 20°C est égale à 65,2 mm²/s.

### Exemple 2 Préparation et analyse d'une composition exempte de glycérine

On procède à l'identique de l'Exemple 1, mais en remplaçant la glycérine d'origine végétale par 1kg de 1,3-butanediol (fourni par Kyowa) selon l'Exemple 1. On procède alors à une mesure de la viscosité dans les mêmes conditions que dans l'exemple 1.

### Résultats :

La viscosité à 20°C est égale à 101,2 mm²/s.

### Exemple 3 (comparatif): Evaluation de l'efficacité de différents e-liquides

On procède à l'identique de l'Exemple 1, mais en remplaçant le 1,3-butanediol par du propylène glycol synthétique fourni par la société Dow sous la référence Dow® Propylene glycol, grade USP. On procède alors à une mesure de la viscosité à 20°C.

### Résultats :

La viscosité à 20°C est égale à 59,2 mm²/s.

En conclusion, on peut constater que l'on peut obtenir des e-liquides à base de 1,3-butanediol, associé ou pas à la glycérine (Exemples 1 et 2) présentant des viscosités tout à fait comparables à celle d'un e-liquide conventionnel (Exemple 3).

### Exemple 4a) : Evaluation de l'efficacité de différents e-liquides

Les compositions de e-liquides préparées dans les Exemples 1 à 3 sont évaluées par un panel entraîné de 10 personnes équipées d'une cigarette de marque Joytech™ et de modèle eCab™ (modèle décembre 2013). Chaque réservoir est rempli d'une quantité identique de e-liquide (1 ml). Aussi, chaque paneliste effectue en aveugle un essai sur la base de 8 bouffées successives espacées de 20 secondes et induites chacune par un chauffage de 2 secondes. L'évaluation repose sur la notation, sur une échelle de 1 à 10, des critères de densité de vapeur et de puissance aromatique ressentie. Le passage d'un produit à l'autre est effectué par chaque paneliste de la façon suivante : 5 minutes après la dernière aspiration, le panéliste rince sa bouche à l'aide de 2 verres d'eau de 100 ml puis se désaltère avec 50 ml d'eau. Un temps de repos entre chaque évaluation est fixé à 10 minutes.

Les résultats moyennés obtenus sont rassemblés dans le tableau suivant :

| Produit | Densité de vapeur (note 1 à 10) | Puissance aromatique (note 1 à 10) |
|---|---|---|
| Exemple 1 | 7,2 ± 1,3 | 6,7 ± 1,5 |
| Exemple 2 | 7,9 ± 1,2 | 7,8 ± 1,2 |
| Exemple 3 | 6,8 ± 1,1 | 5,4, ± 1,1 |

Il apparaît clairement que le 1,3-butanediol associé à la glycérine d'origine végétale (Exemple 1) ou non associé à la glycérine (Exemple 2) permet de produire un e-liquide présentant une densité de vapeur tout à fait comparable, voire supérieure à celle d'un e-liquide conventionnel (Exemple 3). Les e-liquides à base de 1,3-butanediol permettent l'obtention d'une puissance aromatique supérieure à celle obtenue avec un e-liquide conventionnel (exemple 3).

### Exemple 4b) : Formulations de e-liquides

Dans un mélangeur en verre équipé d'une agitation mécanique on mélange précisément 5,50 kg de propylène glycol d'origine végétale (colza) commercialisé par la société Oléon sous la référence Radianol® 4710, grade Pharmacopée USP), 4,00 kg de glycérine végétale (commercialisée par la société Oléon sous la référence Glycérine 4810, grade Pharmacopées USP et PE), 50 g d'eau osmosée et 162,6 g de nicotine végétale (commercialisée par la société Alchem International sous la référence Nicotine Free Base, Pharmaceutical grade Nicotine >99%). On maintient l'agitation du mélange (50 tours/minute) pendant 20 minutes. On obtient alors le produit A.

On procède de façon identique au produit A pour préparer le produit B en remplaçant le propylène glycol et la glycérine végétale par 9 837,4 g de 1,3-butanediol fourni par la société Kyowa.

### Exemple 5 : Influence de la nature du solvant sur les propriétés sensorielles du e-liquide et sur la facilité d'aspiration du e-liquide

Les compositions de e-liquides, Produits A et B et C (ce dernier correspondant à du 1,3 butanediol sans nicotine ajoutée), préparées à l'exemple 4b), sont évaluées par un panel entraîné de 40 personnes (sexe masculin, âge compris entre 25 et 49 ans), équipées d'une cigarette de marque Joytech™ et de modèle eCab™ (modèle décembre 2013). Chaque réservoir est rempli d'une quantité identique de e-liquide (1 ml).

Aussi, chaque panéliste effectue en aveugle un essai sur la base de 8 bouffées successives espacées de 20 secondes et induites chacune par un chauffage de 2 secondes. Le passage d'un produit à l'autre est effectué par chaque paneliste de la façon suivante : 5 minutes après la dernière aspiration, le panéliste rince sa bouche à l'aide de 2 verres d'eau de 100 ml puis se désaltère avec 50 ml d'eau. Un temps de repos entre chaque évaluation est fixé à 10 minutes. L'évaluation repose sur la notation, sur une échelle de 1 à 10, les critères suivants :
1) le ressenti du « throat hit », c'est-à-dire l'effet de picotement interne de la gorge classiquement obtenu lorsqu'un fumeur aspire une bouffée de cigarette, qui est également ressenti lorsqu'un utilisateur de cigarette électronique aspire une vapeur de e-liquide riche en nicotine,
2) la facilité d'aspiration de la vapeur du e-liquide.

Les résultats moyennés obtenus sont rassemblés dans le tableau suivant :

| Produit | Ressenti du « Throat Hit » (note 1 à 10) | Facilité d'aspiration (note 1 à 10) |
|---|---|---|
| Produit A | 6,4 ± 1,2 | 6,9 ± 1,4 |
| Produit B | 8,9 ± 0,9 | 6,7 ± 1,1 |
| Produit C | 8,1 ± 0,8 | 6,3 ± 1,2 |

Il apparaît clairement que le 1,3-butanediol associé à la nicotine (produit B) induit un « hit throat » bien supérieur à un produit classique constitué de glycérine, de propylène glycol, d'eau et de nicotine (Produit A). Enfin, il est très intéressant de souligner que le 1,3-butanediol seul (produit C) induit un « hit throat » très significatif en absence totale de nicotine et significativement supérieur au produit A correspondant à un e-liquide conventionnel.

Relativement à la facilité d'aspiration de la vapeur, les e-liquides A, B et C présentent des performances sensiblement équivalentes.

### Exemple 6 : Evaluation de la stabilité thermique des formulations à base de 1,3-butanenediol et comparaison avec les formulations actuelles à base de propylène glycol et de glycérine

La stabilité thermique du 1,3-butanediol et de son mélange avec la nicotine a été évaluée et comparée à des compositions actuelles à base de propylène glycol (PG) et de glycérine végétale (GV). La stabilité des produits et l'interaction possible entre la nicotine et 1,3-butanediol ont été évaluées par analyses thermogravimétrique (ATG) et thermique différentielle(ATD) sur un appareil Q600 TA Instrument. L'analyse thermogravimétrique est une méthode d'analyse thermique dans laquelle les changements des propriétés physiques et chimiques des matériaux sont mesurés en fonction de la température. L'ATG peut fournir des informations sur les phénomènes physiques, tels que la vaporisation ou la combustion. De même, on peut obtenir des informations sur les phénomènes chimiques comme la déshydratation ou la décomposition. Par analyse thermique différentielle (ATD), le matériau d'étude et une référence inerte subissent des cycles thermiques identiques. Pendant l'analyse, la différence de température entre l'échantillon et la référence est enregistrée. Ces différences de températures suivies en fonction du temps par rapport à la référence inerte, nous renseignent sur les phénomènes thermiques subis par l'échantillon, soit exothermiques ou endothermiques. L'analyse ATD donne par intégration de la courbe du Heat Flow les valeurs d'enthalpies qui sont dites endothermiques lorsqu'il y a une absorption d'énergie, ou exothermiques lorsqu'il y a une libération d'énergie sous la forme de chaleur. Le caractère endothermique est lié à un changement d'état du composé (ex. vaporisation) alors que le caractère exothermique est induit par des réactions de décomposition ou des réactions chimiques intra et intermoléculaires. L'analyseur thermogravimétrique Q600 de TA INSTRUMENT a été utilisé pour déterminer conjointement les températures de vaporisation des produits, les valeurs d'enthalpie et le taux de produits non vaporisés à 350°C. Les conditions d'analyse sont les suivantes :
- prise d'essai : 50 mg
- creuset ouvert sous flux d'air : 100 ml/min
- rampe de chauffage : 10°C/min
- domaine de température : 25 à 350°C (conditions de température représentatives d'une utilisation normale ainsi que de mésusage de cigarette électronique). Entre chaque analyse, l'appareil est nettoyé par chauffage à 600°C sous flux d'air, pour éliminer toute trace de résidu non vaporisé et déposé sur le creuset, les bras de balance ou les parois du four.

Les résultats obtenus sont présentés dans le tableau ci-après :

| Produit | ATD Température Vaporisation (°C) (1) | ATD Température transformation (°C) (2) | ATG Résidu non évaporé à 250°C (%) |
|---|---|---|---|
| 1,3 butanediol | 171 | ND | ND |
| Nicotine | 190 | ND | 1,08 |
| 1,3 butanediol + Nicotine 10 mg/ml | 175 | ND | ND |
| 20% GV + 80% PG | 162-209 | **301** | **0,65** |
| 40% GV + 60% PG | 162-233 | **303** | **1,64** |
| 50% GV + 50% PG | 166-239 | **304** | **0,85** |
| 60% GV + 40% PG | 162-243 | **303** | **1,19** |

| | | | |
|---|---|---|---|
| (1) pic endothermique (2) pic exothermique (réaction et/ou décomposition) | | | |

### Conclusions :

- le 1,3 butanediol se vaporise à une température de 171°C sans aucune décomposition thermique ;
- la nicotine se vaporise à une température de 190°C en produisant un résidu après évaporation de 1,08% lié à la présence dans la nicotine d'un composé lourd non vaporisable ;
- en mélange, le 1,3-butanediol et la nicotine se vaporisent simultanément à 175°C (1 seul pic de vaporisation observé en ATD) sans donner lieu à une décomposition et/ou sans réagir entre eux.

Par conséquent, une formulation à base de 1,3-butanediol et de nicotine est parfaitement adaptée pour assurer une délivrance constante de la nicotine. En effet, une vaporisation trop différente entre le solvant et la nicotine entraînerait au cours du temps (c'est-à-dire au cours de l'utilisation de la cigarette électronqiue) un appauvrissement du mélange en composé le plus volatil et un enrichissement en composé le plus lourd. Dans ces conditions, la concentration de la nicotine dans l'aérosol ne pourrait être constante au cours du temps. Enfin, le 1,3-butanediol est un solvant de la nicotine thermiquement stable, qui se vaporise sans interagir avec la nicotine.
- L'ensemble des formulations à base de propylène glycol conduit à l'apparition en ATD de pics exothermiques caractéristiques de réactions intermoléculaires. Il y a donc interaction chimique à chaud (301-304°C) entre les constituants de la formulation ;
- L'ensemble des formulations à base de propylène glycol conduit à la présence de résidus non vaporisables à 350°C correspondant à la formation de composés secondaires lourds dont la teneur est comprise entre 0,6 et 1,7% environ;

La stabilité thermique du 1,3-butanediol ainsi que son absence de réaction à chaud avec la nicotine, démontrent son intérêt comme base de formulation des liquides destinés aux vaporisateurs personnels. En effet, ce type de formulation ne génère à chaud, aucun composé toxique volatil tel que le formaldéhyde, l'acétaldéhyde, l'acroléine ou encore des composés secondaires lourds. Ce qui n'est pas le cas des formulations actuelles à base de propylène glycol et de glycérine, pour lesquelles se pose un véritable problème technique et d'innocuité *(**1.***Goniewicz & coll. Levels of Selected Carcinogens and Toxicants in Vapour from Electronic Cigarettes - TC Online First, publié le 6 Mars 2013 sous 10.1136/tobaccocontrol-20 2012-050859*). **2.*** Jensen, B.S. Wentai Luo, J. Pankow, R. M. Strongin, D. H. Peyton. Hidden Formaldehyde in E-cigarettes Aerosols. New England Journal of Medicine, 372;4, pp 392-3,93, January 35 22,2015*). **3.*** K. Bekki, S. Uchiyama, K Ohta, Y.Inaba, H. Nakagome, N. Kunugita. Carbonyl Compounds, Generated from Electronic Cigarettes. Int. J. Environ. Res.Public Health 2014, 11, 11192-11200*)*
Wentai Luo, J. Pankow, R. M. Strongin, D. H. Peyton. Hidden Formaldehyde in E-cigarettes Aerosols. New England Journal of Medicine, 372;4, pp 392-3,93, January 35 22,2015*). **3.*** K. Bekki, S. Uchiyama, K Ohta, Y.Inaba, H. Nakagome, N. Kunugita. Carbonyl Compounds,Generated from Electronic Cigarettes. Int. J. Environ. Res.Public Health 2014, 11, 11192-11200*)*

## Revendications

1. Composition de liquide pour cigarette électronique comprenant un polyol, ainsi qu'au moins un composé choisi parmi : la nicotine, un substitut de nicotine et un arôme, **caractérisée en ce que** le polyol est en C4-C8 ayant 2 groupes hydroxyles, et **en ce que** ladite composition ne renferme pas d'eau.

2. Composition selon la revendication 1, **caractérisée en ce que** ledit arôme est à une concentration allant de 1 à 8% par rapport au poids total de ladite composition.

3. Composition selon l'une quelconque des revendications précédentes, **caractérisée en ce qu'**elle est exempte de glycérine.

4. Composition selon l'une quelconque des revendications précédentes, **caractérisée en ce que** le polyol est un composé appartenant au groupe constitué par le 1,2-butanediol, le 1,3-butanediol, le 1,4-butanediol, le 1,2-pentanediol, le 1,2-hexanediol, le 1,2-heptanediol, le 1,2-octanediol et leurs mélanges.

5. Cigarette électronique renfermant une composition selon l'une quelconque des revendications 1 à 4.

6. Utilisation d'une composition selon l'une quelconque des revendications 1 à 4 comme liquide de cigarette électronique.

7. Utilisation d'un polyol en C4-C8 ayant 2 groupes hydroxyles dans un liquide de cigarette électronique renfermant ou non de la nicotine, pour améliorer le picotement de la gorge ressenti par un utilisateur dudit liquide et/ou la facilité d'aspiration de la vapeur produite par ledit liquide.

8. Utilisation d'un polyol en C4-C8 ayant 2 groupes hydroxyles dans un liquide de cigarette électronique renfermant de la nicotine, pour améliorer la biodisponibilité de la nicotine.

9. Utilisation d'un polyol en C4-C8 ayant 2 groupes hydroxyles dans un liquide de cigarette électronique pour renforcer la puissance aromatique.

10. Utilisation d'un polyol en C4-C8 ayant 2 groupes hydroxyles dans un liquide de cigarette électronique pour limiter ou pour supprimer la formation de coproduits de thermolyse.

11. Utilisation d'un polyol en C4-C8 ayant 2 groupes hydroxyles dans un liquide de cigarette électronique pour augmenter la densité de vapeur.

## Patentansprüche

1. Flüssigkeitszusammensetzung für eine elektrische Zigarette, umfassend ein Polyol und wenigstens eine Verbindung ausgewählt aus Nikotin, einem Nikotinsubstitut und einem Aroma, **dadurch gekennzeichnet, dass** das Polyol vier bis acht Kohlenstoffatome und zwei Hydroxylgruppen umfasst und die Zusammensetzung kein Wasser umfasst.

2. Zusammensetzung nach Anspruch 1, **dadurch gekennzeichnet, dass** das Aroma mit einem Gehalt von 1 bis 8 % bezogen auf das Gesamtgewicht der Zusammensetzung vorhanden ist.

3. Zusammensetzung nach einem der vorangehenden Ansprüche, **dadurch gekennzeichnet, dass** sie frei von Glycerin ist.

4. Zusammensetzung nach einem der vorangehenden Ansprüche, **dadurch gekennzeichnet, dass** das Polyol eine Verbindung ist, die ausgewählt ist aus der Gruppe bestehend aus 1,2-Butandiol, 1,3-Butandiol, 1,4-Butandiol, 1,2-Pentandiol, 1,2-Hexandiol, 1,2-Heptandiol, 1,2-Octandiol und Mischungen davon.

5. Elektrische Zigarette umfassend eine Zusammensetzung nach einem der Ansprüche 1 bis 4.

6. Verwendung einer Zusammensetzung nach einem der Ansprüche 1 bis 4 als Flüssigkeit für eine elektrische Zigarette.

7. Verwendung eines Polyols umfassend vier bis acht Kohlenstoffatome und zwei Hydroxylgruppen in einer Flüssigkeit für eine elektronische Zigarette, die Nikotin umfassen kann, um das von einem Anwender der Flüssigkeit gefühlte Kribbeln der Kehle und/oder die Leichtigkeit der Aspiration des von der Flüssigkeit erzeugten Dampfes zu verbessern.

8. Verwendung eines Polyols umfassend vier bis acht Kohlenstoffatome und zwei Hydroxylgruppen in einer Flüssigkeit für eine elektronische Zigarette umfassend Nikotin, um die Bioverfügbarkeit des Nikotins zu verbessern.

9. Verwendung eines Polyols umfassend vier bis acht Kohlenstoffatome und zwei Hydroxylgruppen in einer Flüssigkeit für eine elektronische Zigarette, um die Aromaintensität zu verbessern.

10. Verwendung eines Polyols umfassend vier bis acht Kohlenstoffatome und zwei Hydroxylgruppen in einer Flüssigkeit für eine elektronische Zigarette, um die Bildung von Nebenprodukten bei der Thermolyse zu beschränken oder zu unterdrücken.

11. Verwendung eines Polyols umfassend vier bis acht Kohlenstoffatome und zwei Hydroxylgruppen in einer Flüssigkeit für eine elektronische Zigarette, um die Dichte des Dampfes zu steigern.

## Claims

1. Liquid composition for an electronic cigarette comprising a polyol, as well as at least one compound selected from: nicotine, a nicotine substitute and an aroma, **characterized in that** the polyol comprises 4 to 8 carbon atoms and 2 hydroxyl functions, and **in that** said composition does not comprise water.

2. Composition according to claim 1, **characterized in that** said aroma has a concentration from 1 to 8% by weight of said composition.

3. Composition according to any of the preceding claims, **characterized in that** it does not comprise glycerin.

4. Composition according to any of the preceding claims, **characterized in that** the polyol is a compound belonging to the group constituted by 1,2-butane diol, 1,3-butane diol, 1,4-butane diol, 1,2-pentane diol, 1,2-hexane diol, 1,2-heptane diol, 1,2-octane diol and their mixtures.

5. Electronic cigarette comprising a composition according to any one of claims 1 to 4.

6. Use of a composition according to any of claims 1 to 4, as an electronic cigarette e-liquid.

7. Use of a polyol comprising 4 to 8 carbon atoms and 2 hydroxyl functions in an electronic cigarette liquid comprising or not comprising nicotine, in order to improve the tingling of the throat felt by a user of this liquid and/or the ease of inhaling the vapor produced by this liquid.

8. Use of a polyol comprising 4 to 8 carbon atoms and 2 hydroxyl functions in an electronic cigarette liquid comprising nicotine, in order to improve the bioavailability of the nicotine.

9. Use of a polyol comprising 4 to 8 carbon atoms and 2 hydroxyl functions in an electronic cigarette liquid in order to reinforce the aromatic potency.

10. Use of a polyol comprising 4 to 8 carbon atoms and 2 hydroxyl functions in an electronic cigarette liquid in order to limit or eliminate the formation of coproducts of thermolysis.

11. Use of a polyol comprising 4 to 8 carbon atoms and 2 hydroxyl functions in an electronic cigarette liquid in order to augment the vapor density.
